(19) Europäisches Patentamt
European Patent Office
Office européen des brevets

(11) **EP 3 501 529 B1**

(12) **EUROPEAN PATENT SPECIFICATION**

(45) Date of publication and mention
of the grant of the patent:
**19.10.2022 Bulletin 2022/42**

(21) Application number: **18195778.8**

(22) Date of filing: **20.09.2018**

(51) International Patent Classification (IPC):
**A61K 36/287** (2006.01)     **A61K 38/47** (2006.01)
**A61K 31/352** (2006.01)     A61P 17/00 (2006.01)

(52) Cooperative Patent Classification (CPC):
(C-Sets available)
**A61K 36/287; A61K 31/352; A61K 38/47;
A61P 17/00;** A61K 2236/00           (Cont.)

(54) **METHOD FOR PREPARING AN EXTRACT OF CHRYSANTHEMUM MORIFOLIUM WITH AN EFFECT OF TREATING SKIN DISEASES, EXTRACT OF CHRYSANTHEMUM MORIFOLIUM WITH AN EFFECT OF TREATING SKIN DISEASES AND PHARMACEUTICAL COMPOSITION CONTAINING THE EXTRACT**

VERFAHREN ZUR HERSTELLUNG EINES EXTRAKTS VON CHRYSANTHEMUM MORIFOLIUM MIT EINER WIRKUNG ZUR BEHANDLUNG VON HAUTERKRANKUNGEN, EXTRAKT VON CHRYSANTHEMUM MORIFOLIUM MIT EINER WIRKUNG ZUR BEHANDLUNG VON HAUTERKRANKUNGEN UND PHARMAZEUTISCHE ZUSAMMENSETZUNG MIT DEM EXTRAKT

PROCÉDÉ DE PRÉPARATION D'UN EXTRAIT DE CHRYSANTHEMUM MORIFOLIUM AVEC UN EFFET DE TRAITEMENT DES MALADIES DE LA PEAU, EXTRAIT DE CHRYSANTHEMUM MORIFOLIUM AVEC UN EFFET DE TRAITEMENT DES MALADIES DE LA PEAU ET COMPOSITION PHARMACEUTIQUE CONTENANT L'EXTRAIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2017 TW 106145261**

(43) Date of publication of application:
**26.06.2019 Bulletin 2019/26**

(73) Proprietor: **Industrial Technology Research Institute**
**31040 Hsinchu (TW)**

(72) Inventors:
• **PAN, I-Horng**
**302 Hsinchu County (TW)**
• **YAO, Hsin-Jan**
**630 Yunlin County (TW)**
• **LU, Chu-Hsun**
**813 Kaohsiung City (TW)**
• **WEN, Shu-Fang**
**308 Hsinchu County (TW)**
• **HSU, Po-Yao**
**400 Taichung City (TW)**

• **LI, Ming-Han**
**105 Taipei City (TW)**
• **CHUANG, Kai-An**
**111 Taipei City (TW)**
• **CHANG, Chih-Hsuan**
**302 Hsinchu County (TW)**

(74) Representative: **Michalski Hüttermann & Partner Patentanwälte mbB**
**Kaistraße 16A**
**40221 Düsseldorf (DE)**

(56) References cited:
**CN-A- 103 610 725     CN-A- 105 175 376**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Xinyan Lu ET AL: "Evaluation of hepatic clearance and drug-drug interactions of luteolin and apigenin by using primary cultured rat hepatocytes", Die Pharmazie, 1 August 2011 (2011-08-01), pages 600-6051517, XP055576594, Germany DOI: 10.1691/ph.2011.1517 Retrieved from the Internet: URL:http://docserver.ingentaconnect.com/de liver/connect/govi/00317144/v66n8/s9.pdf?e xpires=1554207222&id=0000&titleid=75007325 &checksum=506FC31ABD465545ADC5ACD791 D9438A
- LII C K ET AL: "Chrysanthemum morifolium Ramat. reduces the oxidized LDL-induced expression of intercellular adhesion molecule-1 and E-selectin in human umbilical vein endothelial cells", JOURNAL OF ETHNOPHARMACOLOGY, ELSEVIER IRELAND LTD, IE, vol. 128, no. 1, 2 March 2010 (2010-03-02) , pages 213-220, XP026904070, ISSN: 0378-8741, DOI: 10.1016/J.JEP.2010.01.018 [retrieved on 2010-01-15]
- FU YU-JIE ET AL: "Enzyme assisted extraction of luteolin and apigenin from pigeonpea [Cajanuscajan(L.) Millsp.] leaves", FOOD CHEMISTRY, ELSEVIER LTD, NL, vol. 111, no. 2, 7 April 2008 (2008-04-07) , pages 508-512, XP029210977, ISSN: 0308-8146, DOI: 10.1016/J.FOODCHEM.2008.04.003

(52) Cooperative Patent Classification (CPC): (Cont.)

C-Sets
A61K 31/352, A61K 2300/00;
A61K 38/47, A61K 2300/00

**Description**

**TECHNICAL FIELD**

The technical field relates to a preparation method of a plant extract, and in particular it relates to a method for preparing an extract of *Chrysanthemum morifolium* with the effect of treating skin diseases, an extract of *Chrysanthemum morifolium* with the effect of treating skin diseases, and a pharmaceutical composition for treating skin diseases containing this extract. The invention is set out in the appended set of claims.

**BACKGROUND**

Skin diseases are the most common disease in the world, and about one-third of the population has a pathological skin problem during his or her lifetime. In terms of health care spending, skin-related medical costs can reach as high as 25%.

Skin diseases can be divided into four major categories, namely dermatitis (such as allergic and contact), cancer (such as melanoma), immune disease (such as psoriasis), and infectious skin disease (such as bacterial, fungal, and viral infections).

Topical steroids have been used extensively in various skin conditions, especially atopic dermatitis and psoriasis, and in severe dermatitis patients, steroids are used in higher doses. Many compounds classified as steroids, such as betamethasone or prednisolone, are very effective in the treatment of inflammatory diseases. However, long-term use of these compounds may also cause skin atrophy in patients. Patients who suffer from skin atrophy during steroid treatment are generally considered as steroid responders. The influence of skin atrophy on patients already suffering from skin disease symptoms (such as psoriasis patients) is a concern. However, even with normal skin, in the case of long-term use of steroids, there can be side effects including skin damage.

Therefore, development of safe dermatological drugs that can replace steroids, especially botanicals, is an important issue.

In this respect, CN 103 610 725 A describes an extraction method of general flavone in chrysanthemum, and particularly relates to a method for extracting general flavone from chrysanthemum by enzymolysis. According to the method provided by the invention, high-temperature boiling is not required in the whole process, so that steam consumption in production is greatly saved. Meanwhile, low-temperature enzymolysis extraction is carried out to avoid loss of volatile aromatic substances, so that special fragrance of chrysanthemum is basically remained.

Further, CN 105 175 376 A describes a method for extracting luteolin from Laifeng crossostephium leaves. The method includes the step A of enzymolysis, the step B of alcohol leaching, the step C of microwave extraction, the step D of filtering, the step E of concentration and the step F of cooling. The method is low in process condition requirement and reasonable in process, the extraction process is unique, the method is suitable for industrialized production conditions, and the extraction efficiency is high; meanwhile, devices are simple, the manufacturing cost is low, and the method is easy to apply and popularize.

Furthermore, such issues are addressed in Xinyan Lu ET AL, "Evaluation of hepatic clearance and drug-drug interactions of luteolin and apigenin by using primary cultured rat hepatocytes", Die Pharmazie, Germany, doi:10.1691/ph.2011.1517, page 604; LII C K ET AL, "Chrysanthemum morifolium Ramat. reduces the oxidized LDL-induced expression of intercellular adhesion molecule-1 and E-selectin in human umbilical vein endothelial cells", JOURNAL OF ETHNOPHARMACOLOGY, ELSEVIER IRELAND LTD, IE, vol. 128, no. 1, pages 213 - 220, doi:10.1016/J.JEP.2010.01.018, ISSN 0378-8741; and FU YU-JIE ET AL, "Enzyme assisted extraction of luteolin and apigenin from pigeonpea [Cajanuscajan(L.) Millsp.] leaves", FOOD CHEMISTRY, ELSEVIER LTD, NL, (20080407), vol. 111, no. 2, doi:10.1016/J.FOODCHEM.2008.04.003, ISSN 0308-8146, pages 508 - 512.

**SUMMARY**

The invention is described in the independent claims. Preferred embodiments of the invention are described in the sub claims. The present disclosure provides a method for preparing an extract of *Chrysanthemum morifolium* with an effect of treating skin diseases, comprising: (a) performing an extraction procedure on *Chrysanthemum morifolium* with an extraction solvent to obtain an extract solution; and (b) adding at least one glycoside hydrolases to the extract solution to make an enzyme reaction occur to produce a precipitate, wherein the precipitate is the extract of *Chrysanthemum morifolium* with an effect of treating skin diseases.

The present disclosure also provides an extract of *Chrysanthemum morifolium* with an effect of treating skin diseases, which is obtained using the aforementioned method for preparing an extract of *Chrysanthemum morifolium* with an effect of treating skin diseases.

The present disclosure also provides an extract of *Chrysanthemum morifolium* with an effect of treating skin diseases, at least comprising the following two indicator ingredients: luteolin and apigenin, wherein in the extract of *Chrysanthemum morifolium,* the content ratio of the luteolin to the apigenin is about 1:1-30.

The present disclosure further provides a pharmaceutical composition for treating skin diseases, comprising: any aforementioned extract of *Chrysanthemum morifolium* with an effect of treating skin diseases; and a pharmaceutically acceptable vehicle, carrier or salt.

A detailed description is given in the following embodiments with reference to the accompanying drawings.

## BRIEF DESCRIPTION OF DRAWINGS

The present invention can be more fully understood by reading the subsequent detailed description and examples with references made to the accompanying drawings, wherein:

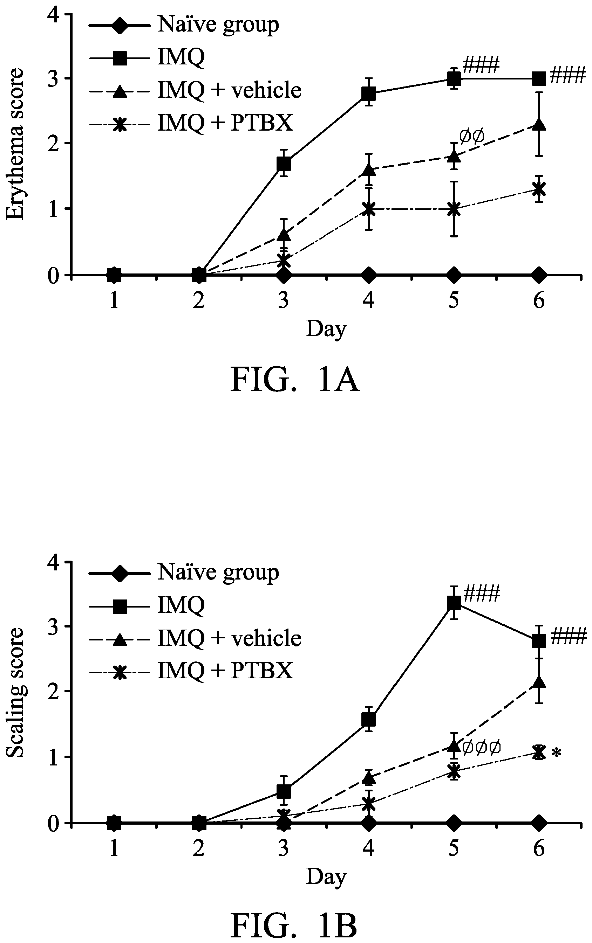

FIG. 1A shows the erythema scores of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. ###: *p<0.001,* as compared to the naive group; øø: *p<0.01,* as compared to the control group. T-test;

FIG. 1B shows the scaling scores of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. ###: *p<0.001,* as compared to the naive group; øøø: *p<0.001,* as compared to the control group. *: *p<0.05,* as compared to the imiquimod (IMQ) + vehicle group. T-test;

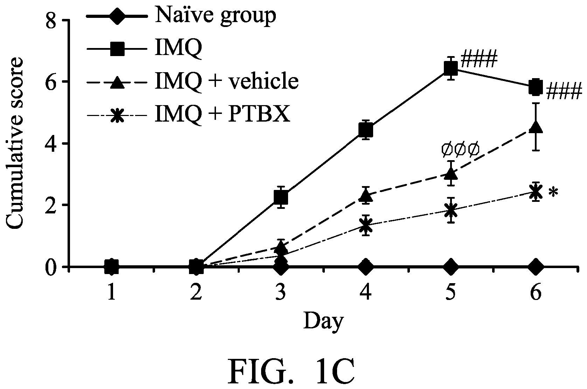

FIG. 1C shows the cumulative score of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. ###: *p<0.001,* as compared to the naive group; øøø: *p<0.001,* as compared to the control group. *: *p<0.05,* as compared to the imiquimod (IMQ) + vehicle group. T-test;

FIG. ID shows the photographs and H&E stain results for the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment);

FIG. IE shows the skin thickness of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. ###: *p<0.001,* as compared to the naive group; *: *p<0.05,* as compared to the imiquimod (IMQ) + vehicle group. T-test;

FIG. IF shows the keratin (K14) gene expression levels of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. ###: *p<0.001,* as compared to the naive group; *: *p<0.05,* as compared to the imiquimod (IMQ) + vehicle group. T-test;

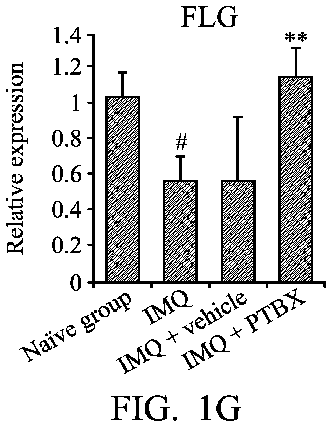

FIG. 1G shows the filaggrin (FLG) gene expression levels of the back skin of the mice of the naive group (no treatment is applied), the control group (50 mg imiquimod (IMQ) cream), the imiquimod (IMQ) + vehicle group (50 mg imiquimod cream + the vehicle of ointment) and the experimental group (50 mg imiquimod cream + PTBX ointment). Mean $\pm$ Standard deviation. #: *p<0.05,* as compared to the naive group; **: *p<0.01,* as compared to the imiquimod (IMQ) + vehicle group. T-test.

## DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The present disclosure provides a method for preparing an extract of *Chrysanthemum morifolium* with the effect of treating skin diseases. The skin diseases described herein have no specific limitation, and may be, for example, dermatitis (such as allergic or contact), immune diseases (such as psoriasis), infectious skin diseases (such as bacterial, fungal, viral infection).

The foregoing method may comprise, but is not limited to the following steps.

First, an extraction procedure is performed on *Chrysanthemum morifolium* with an extraction solvent to obtain an extract solution. The foregoing extract solution may be an extract solution directly obtained from the extraction procedure, or may be a solution formed by re-dissolving a powder which is formed by further drying an extract solution directly obtained from the extraction procedure mentioned above with water or a buffer, and has no specific limitation. Moreover, examples of the buffer may comprise, but is not limited to, acetate buffer.

Moreover, examples of the extraction solvent mentioned above may include water and alcohols, but they are not limited thereto. In one embodiment, the extraction solvent may be water.

The temperature of the extraction procedure is about 70-100°C, but it is not limited thereto. In one embodiment, the temperature of the extraction procedure is about 80-95°C. Moreover, the time required for the extraction procedure may be about 1-3 hours, but it is not limited thereto. In one embodiment, the time required for the extraction procedure may be about 1-2 hours.

Next, at least one glycoside hydrolase is added to the extract solution to make an enzyme reaction occur to produce a precipitate, and the obtained precipitate is the extract of *Chrysanthemum morifolium* with the effect of treating skin diseases.

The weight ratio of the foregoing glycoside hydrolase to the foregoing *Chrysanthemum morifolium* may be about 1:200-10000, such as 1:3000-8000, 1:1500-4000, 1:750-2000, 1:300-800, but it is not limited thereto. In one embodiment, the weight ratio of the foregoing glycoside hydrolase to the foregoing *Chrysanthemum morifolium* may be about 1:3000-8000. In another embodiment, the weight ratio of the foregoing glycoside hydrolase to the foregoing *Chrysanthemum morifolium* may be about 1:1500-4000. In yet another embodiment, the weight ratio of the foregoing glycoside hydrolase to the foregoing *Chrysanthemum morifolium* may be about 1:750-2000. Furthermore, in another embodiment, the weight ratio of the foregoing glycoside hydrolase to the foregoing *Chrysanthemum morifolium* may be about 1:300-800.

Moreover, the temperature required for the aforementioned enzyme reaction may be about 25-45°C, such as 25°C, 30°C, 35°C, 36°C, 37°C, 37.5°C, 38°C, 39°C, 40°C, but it is not limited thereto. In one embodiment, the temperature of the aforementioned enzyme reaction may be about 37°C. In addition, the time required for the aforementioned enzyme reaction may be about 5-30 hours, such as 5-25 hours, 10-24 hours, but it is not limited thereto. In one embodiment, the time required for the aforementioned enzyme reaction may be about 16-24 hours.

Examples of the foregoing glycoside hydrolase may include, glucosidase, galactosidase, glucuronidase, and any combinations thereof. The foregoing glucosidase may comprise α-glucosidase, β-glucosidase, or a combination thereof. The foregoing galactosidase may comprise α-galactosidase, β-galactosidase, or a combination thereof. Furthermore, the foregoing glucuronidase may comprise α-glucuronidase, β-glucuronidase, or a combination thereof.

In one embodiment, the foregoing at least one glycoside hydrolase may be glucosidase, and in this embodiment, the foregoing glucosidase is β-glucosidase. Moreover, in this embodiment, the weight ratio of the foregoing β-glucosidase to the foregoing *Chrysanthemum morifolium* may be about 1:200-10000, such as 1:3000-8000, 1:1500-4000, 1:750-2000, 1:300-800, but it is not limited thereto. In one embodiment, the weight ratio of the foregoing β-glucosidase to the foregoing *Chrysanthemum morifolium* may be about 1:3000-8000. In another embodiment, the weight ratio of the foregoing β-glucosidase to the foregoing *Chrysanthemum morifolium* may be about 1:1500-4000. In yet another embodiment, the weight ratio of the foregoing β-glucosidase to the foregoing *Chrysanthemum morifolium* may be about 1:1500. Moreover, in another embodiment, the weight ratio of the foregoing β-glucosidase to the foregoing *Chrysanthemum morifolium* may be about 1:750-2000. In yet another embodiment, the weight ratio of the foregoing β-glucosidase to the foregoing *Chrysanthemum morifolium* may be about 1:300-800.

In one embodiment, the foregoing at least one glycoside hydrolase may be glucosidase and glucuronidase. In this embodiment, the weight ratio of the glucosidase to the glucuronidase may be about 1-2:1-2, such as 1:1, 1.5:1, 2:1, 1:1.5, 1:2, etc., but it is not limited thereto. Moreover, in this embodiment, the weight ratio of the glucosidase and the glucuronidase to the foregoing *Chrysanthemum morifolium* may be about 1:200-10000, such as 1:3000-8000, 1:1500-4000, 1:750-2000, 1:300-800, but it is not limited thereto. In one embodiment, the weight ratio of the glucosidase and the glucuronidase to the foregoing *Chrysanthemum morifolium* may be about 1:1500-4000.

In addition, in the method for preparing an extract of *Chrysanthemum morifolium* with the effect of treating skin diseases of the present disclosure mentioned above, in one embodiment, water is used to perform the extraction procedure on the *Chrysanthemum morifolium* to obtain a water extract of *Chrysanthemum morifolium*.

Moreover, in this embodiment, the temperature of the extraction procedure mentioned above may be about 70-100°C, and time required for the extraction procedure mentioned above may be about 1-3 hours.

Furthermore, in the foregoing embodiment in which water is used as the extraction solvent, in one specific embodiment, in the step of adding at least one glycoside hydrolase to the extract solution to make an enzyme reaction occur to produce a precipitate, the at least one glycoside hydrolase which is used is glucosidase. This glucosidase may be β-glucosidase. Furthermore, the temperature of the foregoing enzyme reaction may be 25-45°C, such as 25°C, 30°C, 35°C, 36°C, 37°C, 37.5°C, 38 °C, 39°C, 40°C, but it is not limited thereto, and in one specific embodiment, the temperature of the aforementioned enzyme reaction may be about 37°C. In addition, the time required for the foregoing enzyme reaction may be about 5-30 hours, such as 5-25 hours, 16-24 hours, but it is not limited thereto, and in one specific embodiment, the time required for the foregoing enzyme reaction may be about 16-24 hours. When the at least one glycoside hydrolase which is used is β-glucosidase, the weight ratio of the β-glucosidase to the *Chrysanthemum mori-*

*folium* may be about 1:300-10000, such as 1:3000-8000, 1:1500-4000, 1:750-2000, 1:400-800, but it is not limited thereto.

In addition, in the foregoing embodiment in which water is used as the extraction solvent, in another specific embodiment, in the step of adding at least one glycoside hydrolase to the extract solution to make an enzyme reaction occur to produce a precipitate, the at least one glycoside hydrolase which is used is glucosidase and glucuronidase. This glucosidase may be β-glucosidase while this glucuronidase may be β-glucuronidase. Furthermore, the temperature of the foregoing enzyme reaction may be 35-40°C, such as 35°C, 36°C, 37°C, 37.5°C, 38°C, 39°C, 40°C, but it is not limited thereto, and in one specific embodiment, the temperature of the aforementioned enzyme reaction may be about 37°C. In addition, the time required for the foregoing enzyme reaction may be about 5-30 hours, such as 5-25 hours, 16-24 hours, but it is not limited thereto, and in one specific embodiment, the time required for the foregoing enzyme reaction may be about 16-24 hours. When the at least one glycoside hydrolase which is used is β-glucosidase and β-glucuronidase, the weight ratio of the β-glucosidase and β-glucuronidase to the *Chrysanthemum morifolium* may be about 1:500-5000, such as 1:1500-4000, but it is not limited thereto. Furthermore, the weight ratio of the β-glucosidase to β-glucuronidase may be about 1:2-1:2, such as 1:1. 1.5:1, 2:1, 1:1.5, 1:2, etc.

With regard to the method for preparing an extract of *Chrysanthemum morifolium* with the effect of treating skin diseases of the present disclosure mentioned above, in another embodiment, in the step of adding at least one glycoside hydrolase to the extract solution to make an enzyme reaction occur to produce a precipitate, after the enzyme reaction, a cooling procedure may be further performed to promote production of the precipitate. The temperature of the cooling procedure may be about 2-15°C, such as 4°C, but it is not limited thereto. The time required for the cooling procedure may be about 2-24 hours, such as about 24 hours.

In another embodiment, the method for preparing an extract of *Chrysanthemum morifolium* with the effect of treating skin diseases of the present disclosure mentioned above may further comprise performing a washing procedure on the precipitate after the step of adding at least one glycoside hydrolase to the extract solution to make an enzyme reaction occur to produce a precipitate.

The aforementioned washing procedure may comprise the following steps, but it is not limited thereto.

First, the foregoing precipitate is re-dissolved with an alcohol solvent to form a mixture solution, and the mixture solution comprises a solution part and an insoluble matter. In one embodiment, examples of the alcohol solvent mentioned above may include, but are not limited to, methanol and ethanol. In one embodiment, the alcohol solvent mentioned above may be methanol.

After that, the solution part is concentrated and dried to obtain a washed precipitate. This precipitate has the effect of treating skin diseases.

By employing any method for preparing an extract of *Chrysanthemum morifolium* with the effect of treating skin diseases of the present disclosure mentioned above, after an extract of *Chrysanthemum morifolium* is subjected an enzyme treatment and that results in a bio-conversion, the active ingredients of the extract of *Chrysanthemum morifolium* can be raised, and the solubility of the ingredients of the extract of *Chrysanthemum morifolium* to the solvent can be changed to achieve the purpose of precipitation purification.

Furthermore, by using any method for preparing an extract of *Chrysanthemum morifolium* with the effect of treating skin diseases of the present disclosure mentioned above, the total content of two indicator ingredients of the extract of *Chrysanthemum morifolium,* luteolin and apigenin, can be greatly increased, and a high-purity extract of *Chrysanthemum morifolium* can be obtained only through a one-step enzyme treatment to obtain the effect of one-step purification. In the extract of *Chrysanthemum morifolium* obtained by any method for preparing an extract of *Chrysanthemum morifolium* with the effect of treating skin diseases of the present disclosure mentioned above, the sum of the contents of luteolin and apigenin may be 15-85 wt%,

The content ratio of the luteolin to the apigenin may be about 1:1-30, but it is not limited thereto. In one embodiment, the content ratio of the luteolin to the apigenin may be about 1:1-30, such as 1:1-25, but it is not limited thereto.

Accordingly, based on the foregoing, it can be known that the present disclosure also can provide an extract of *Chrysanthemum morifolium* with the effect of treating skin diseases, which can be obtained by any method for preparing an extract of *Chrysanthemum morifolium* with the effect of treating skin diseases of the present disclosure mentioned above.

The extract of *Chrysanthemum morifolium* obtained by the method for preparing an extract of *Chrysanthemum morifolium* with the effect of treating skin diseases of the present disclosure mentioned above may at least comprise, but is not limited to, two indicator ingredients, luteolin and apigenin. In the extract of *Chrysanthemum morifolium* mentioned above, the sum of the contents of luteolin and apigenin may be 15-85 wt%,

In the extract of *Chrysanthemum morifolium* obtained by the method for preparing an extract of *Chrysanthemum morifolium* with the effect of treating skin diseases of the present disclosure mentioned above, the content ratio of the luteolin to the apigenin may be about 1:1-30, but it is not limited thereto. In one embodiment, the content ratio of the luteolin to the apigenin may be about 1:1-30, such as 1:1-25, but it is not limited thereto.

Furthermore, the present disclosure may also provide another an extract of *Chrysanthemum morifolium* with the effect of treating skin diseases which may at least comprise, but is not limited to, two indicator ingredients, luteolin and apigenin while the content ratio of the luteolin to the apigenin may be about 1:1-30, but it is not limited thereto.

In one embodiment, the content ratio of the luteolin to the apigenin may be about 1:1-30, such as 1:1-25, but it is not limited thereto.

In addition, the present disclosure further provides a pharmaceutical composition for treating skin diseases. The pharmaceutical composition for treating skin diseases of the present disclosure may comprise, but is not limited to any aforementioned extract of *Chrysanthemum morifolium* with the effect of treating skin diseases of the present disclosure and a pharmaceutically acceptable vehicle, carrier or salt.

The pharmaceutically acceptable vehicles may act as a dilutant, dispersant or carrier for the active ingredient. The pharmaceutically acceptable vehicle may comprise materials commonly employed in skin care products such as water, liquid or solid emollients, silicone oils, emulsifiers, solvents, humectants, thickeners, powders, propellants and the like.

The vehicle may be formed from 80%- 99.9 wt%, preferably from 90-99% by weight of the compositions mentioned above, and can, in the absence of other adjuncts, form the balance of the compositions.

Moreover, other specific ingredients which benefit skin, such as sunscreens, skin-lightening agents, and skin tanning agents may be also included in the compositions mentioned above. The vehicle may also further include adjuncts such as antioxidants, perfumes, opacifiers, preservatives, colourants and buffers.

In addition, in one embodiment, all of the compositions mentioned may be manufactured as a skin spreading form, including, but not limited to creams, ointments, gels, sprays, lotions, skin tonics, shampoos or mousses, etc. Skin sprays are generally composed of aerosolized copolymers, such as polyvinylpyrrolidone, vinyl acetate and the like, and may also function as a setting lotion. Skin gel preparations are similar to sprays in composition, but are in gel and alcohol free form, and can coat the skin. A skin mousse is foam released under pressure from an aerosolized can. Skin creams may be a hydrophobic or hydrophilic cream, ointment, gel, emollient, spray, lotion, skin tonic, shampoo or mousse, suitably with additional ingredients suitable for use in skin cream of types known in the art, and such further ingredients can include petrolatum, waxes, lanolin, silicone, liposomes, vegetable, mineral oils, plasticizers, fragrances, preservatives, a penetration enhancing agent, a pH adjusting agent or other suitable ingredients for skin creams. Such ingredients can moisturize skin, stabilize the active compound, increase the composition-skin contact to further raise local concentration and control the composition release.

The pharmaceutically acceptable carrier mentioned above may comprise, but is not limited to, a solvent, a dispersion medium, a coating, an antibacterial and antifungal agent, or an isotonic and absorption delaying agent, etc. which is suitable for pharmaceutical administration. The pharmaceutical composition can be formulated into dosage forms for different administration routes utilizing conventional methods.

Furthermore, the pharmaceutically acceptable salt mentioned above may include, but is not limited to, salts including inorganic cation, such as alkali metal salts such as sodium salt, potassium salt or amine salt, such as alkaline-earth metal salt such as magnesium salt or calcium salt, such as the salt containing bivalent or quadrivalent cation such as zinc salt, aluminum salt or zirconium salt. In addition, the pharmaceutically acceptable salt may also be organic salt, such as dicyclohexylamine salt, methyl-D-glucamine, and amino acid salt such as arginine, lysine, histidine, or glutamine.

The pharmaceutical composition of the present disclosure may be administered orally, parenterally by an inhalation spray, or via an implanted reservoir. The parenteral methods may comprise smearing affected regions, subcutaneous, intracutaneous, intravenous, intramuscular, intra-articular, intra-arterial, intrasynovial, intrasternal, intrathecal, and intraleaional injection, as well as infusion techniques.

An oral composition may include, but is not limited to, tablets, capsules, emulsions, and aqueous suspensions, dispersions and solutions.

Forms of topical compositions for smearing may comprise ointments, creams, solutions, gels, etc. but they are not limited thereto.

The pharmaceutical composition for treating skin diseases of the present disclosure may be a topical dosage form or a systemic dosage form, but it is not limited thereto. In one embodiment, the pharmaceutical composition for treating skin diseases of the present disclosure may be a topical dosage form, and examples of this topical dosage form may include, but are not limited to, ointments, creams, solutions, and gels.

Any foregoing pharmaceutical composition for treating skin diseases of the present disclosure can be used to treat various skin diseases, and has no specific limitation. Moreover, all of the foregoing pharmaceutical compositions for treating skin diseases of the present disclosure have excellent effects for treatment of various skin diseases, and they have excellent effects particularly for treating and/or alleviating skin inflammation.

In one embodiment, any foregoing pharmaceutical composition for treating skin diseases of the present disclosure can be used to treat allergic or contact dermatitis. In another embodiment, any foregoing pharmaceutical composition for treating skin diseases of the present disclosure can be used to treat autoimmune skin diseases, such as psoriasis.

EXAMPLES

Example 1

Preparation of crude extract PTB1

*Chrysanthemum morifolium* and 15 times its weight in water were mixed to form a mixture. The mixture was heated to ebullition (about 90-100°C) to perform a heating extraction for 1 hour, and then solid residues therein were removed to obtain an extract solution. A vacuum concentrating and drying procedure was performed on the extract solution to obtain a crude extract PTB1. The ratio of the weight of the obtained crude extract PTB1 to the weight of the original herb material was about 1:1.5-4.

Example 2

Preparation of enzyme-treated extract PTBX

*Chrysanthemum morifolium* and 15 times its weight in water were mixed to form a mixture. The mixture was heated to ebullition (about 90-100°C) to perform a heating extraction for 1 hour, and then solid residues therein were removed to obtain an extract solution.

After the extract solution was cooled to room temperature, an enzyme, β-glucosidase, was added therein, and the ratio of the weight of the added enzyme to the weight of the original herb material was 1:1500. Next, the enzyme-containing extract solution was place in a 37°C incubator to perform reaction for 16-24 hours. After the reaction was completed, the enzyme-reacted extract solution was placed in a 4°C refrigerator for 24 hours to promote the production of precipitate.

Solid-liquid separation was performed on the solution to take the precipitate out and the precipitate was re-dissolved with methanol to from a mixture solution, and the mixture solution contained a solution part and an insoluble matter. After that, the solution part was taken out and a vacuum concentrating and drying procedure was performed thereon to obtain an enzyme-treated extract PTBX.

Example 3

1. Preparation of PTBX ointment

A mixture of PTBX dry powder (1 g) and 95% ethanol (1.4 g), polyethylene glycol 400 (10 g), polyethylene glycol 4000 (4.5 g), ethoxylated hydrogenated castor oil (0.15 g) and high-purity deionized water (2.95 ml) was heated to 60°C and stirred until it was uniformly mixed to make an external ointment dosage form, wherein 95% ethanol, polyethylene glycol 400, polyethylene glycol 4000, ethoxylated hydrogenated castor oil and high-purity deionized water were ingredients of vehicle.

2. Preparation of simple vehicle

A mixture of 95% ethanol (1.4 g), polyethylene glycol 400 (10 g), polyethylene glycol 4000 (4.5 g), ethoxylated hydrogenated castor oil (0.15 g) and high-purity deionized water (2.95 ml) was heated to 60°C and stirred until it was uniformly mixed.

Example 4

Chemical ingredients change of the extract of *Chrysanthemum morifolium* before and after the enzyme treatment

High-performance liquid chromatography (HPLC) was used to determine the content change of the indicator ingredients in the extract before and after the enzyme treatment.

In this experiment, two flavonoid ingredients, luteolin and apigenin, were selected as the indictor ingredients for the extract of *Chrysanthemum morifolium,* and the content changes of two sugar derivatives, luteolin 7-O-glucoside and apigenin-7-O-glucoside, respectively corresponding to the two indictor ingredients were also observed at the same time, and content analysis for each ingredient were performed by high-performance liquid chromatography.

High-performance liquid chromatography results for the extract of *Chrysanthemum morifolium* before and after the enzyme treatment are shown in Table 1.

Table 1: Ingredient content analysis for the extract of *Chrysanthemum morifolium* before and after the enzyme treatment

| Sample | Luteolin 7-O-glucoside (mg/g) | Apigenin-7-O-glucoside (mg/g) | Luteolin (mg/g) (Indicator ingredient) | Apigenin (mg/g) (Indicator ingredient) | Sum of luteolin and apigenin (mg/g) |
|---|---|---|---|---|---|
| PTB1 (Before the enzyme treatment) | 1.85 | 10.35 | 0.42 | 0.21 | 0.63 |
| PTBX (After the enzyme treatment) | 4.48 | 115.54 | 78.12 | 530.36 | 608.48 |

According to Table 1, it is known that the total content of luteolin and apigenin of the extract of *Chrysanthemum morifolium* was raised from 0.063% (w/w, dry base), before the enzyme treatment, to 60.848% (w/w, dry base), after the enzyme treatment. In other words, the enzyme treatment used in the present disclosure is capable of greatly raising the total content of luteolin and apigenin and achieving the effect of one-step purification.

Example 5

Bio-activity change of the extract of *Chrysanthemum morifolium* before and after the enzyme treatment

1. Evaluation of *in vitro* keratinocyte proliferation inhibiting activity of enzyme-treated extract PTBX

$5 \times 10^3$ HaCaT keratinocytes were inoculated into a 96 well plate, and placed in a 37°C and 5% $CO_2$ incubator for culturing. After culturing for 16 hours, the cell numbers of this time point (To) was used as a reference point for cell proliferation, and the extract of *Chrysanthemum morifolium* from different processes, PTB1 or PTBX was added to the cells for co-culturing. After co-culturing for 48 hours ($T_{48}$), the supernatant in the plate was removed and 50 μl MTT solution (0.5 mg/mL) was added to the cells. After that, the plate was placed in a 37°C and 5% $CO_2$ incubator for culturing 1.5 hours, and then 150 μL DMSO was added to the plate and the plate was shaken for 5 minutes. Then, absorbance at 570 nm was determined by a continuous wavelength microplate analyzer, and cell proliferation activity was calculated using the following formula:

$$\text{Cell proliferation activity} = OD_{T0}/OD_{T48} \times 100.$$

The experimental results are shown in Table 2.

2. Evaluation of *in vitro* inhibition on inflammation induced by lipopolysaccharide (LPS) of enzyme-treated extract PTBX

$5 \times 10^5$ cells/mL of RAW264.7 cells were inoculated into a 96 well plate and cultured at 37°C under 5% $CO_2$ overnight. The supernatant in the plate was removed and lipopolysaccharide (50 ng/mL) and different concentrations of PTB1 or PTBX were added to the cells.
After reacting for 24 hours, the supernatant in the plate was taken and NO content thereof was detected by Griess reagent (Promega, Cat. No. G2930).
Moreover, 50 μl of culturing medium containing MTT (0.5 mg/mL) was added to the cell part in the plate. After that, the plate was placed in a 37°C and 5% $CO_2$ incubator for culturing 15-20 hours, and then 150 μL DMSO was added to the plate and the plate was shaken for 5-10 minutes. Finally, $OD_{570}$ was read by a continuous wavelength microplate analyzer, and cell viability was calculated using the following formula:

$$\text{Cell viability (\%)} = (\text{OD value of the experimental group}/ \text{OD value of the control group}) \times 100.$$

The experimental results are shown in Table 2.

3. Evaluation of *in vitro* inhibition activity on allergy induced by DNFB of enzyme-treated extract PTBX

$2 \times 10^4$ Normal Human Epidermal Keratinocytes (NHEKs) were inoculated into a 96 well plate, and then recombinant TNF-$\alpha$ (100 ng/ml; PeproTech Cat. No.300-01A) was added to the plate for co-culturing with the cells. After co-culturing for 6 hours, DNFB (1 $\mu$M; Sigma Cat. No. D1529) and different concentrations of PTB1 or PTBX were added to the plate and the culturing was continued for 48 hours. After that, the supernatant in the plate was collected and Human IL-1$\beta$ DuoSet ELISA (Invitrogen; Cat. No. BMS224) was used according to the manufacturer's recommended procedure to analyze the IL-1$\beta$ content in the supernatant for determination of IL-1$\beta$ expression level of the cells.
The experimental results are shown in Table 2.

4. Evaluation of *in vitro* inhibition activity on pruritus caused by IL-31 of enzyme-treated extract PTBX

$2 \times 10^4$ Normal Human Epidermal Keratinocytes (NHEKs) were inoculated into a 96 well plate, and then TLR1/2 stimulant, Pam3Cys-Ser-(Lys)4 (1 $\mu$g/ml; Abcam; Cat. No. ab14208) was added to the plate for co-culturing with the cells. After co-culturing for 6 hours, recombinant IL-31 (100 ng/ml; PeproTech; Cat. No.200-31) and different concentrations of PTB1 or PTBX were added to the plate and the culturing was continued for 48 hours. After that, the supernatant in the plate was collected and Human CCL2/MCP-1 DuoSet ELISA (R&D; Cat. No. DY279) was used according to the manufacturer's recommended procedure to analyze the MCP-1 content in the supernatant for determination of MCP-1 expression level of the cells.
The experimental results are shown in Table 2.

Table 2: Analysis of *in vitro* bioactivity of the extract of *Chrysanthemum morifolium* before and after the enzyme treatment

| Sample | Total content of the four indicators (%) (Based on solid matter) | Keratoplasia GI$_{50}$ ($\mu$g/ml) | Inflammation induced by LPS IC$_{50}$ ($\mu$g/ml) | Allergy induced by DNFB IC$_{50}$ ($\mu$g/ml) | Pruritus caused by IL-31 IC$_{50}$ ($\mu$g/ml) |
|---|---|---|---|---|---|
| PTB1 (Before the enzyme treatment) | 1.28 | >100 | >100 | >50 | >10 |
| PTBX (After the enzyme treatment) | 72.8 | 25-50 | <25 | 1.3-2.5 | 0.6-1.3 |

According to Table 2, it is known that the effects of enzyme-treated extract PTBX on inhibiting keratinocyte proliferation, inflammation and contact allergy, and relieving itching are all significantly raised.

Example 6

Evaluation of reducing effects on skin inflammation of parapsoriasis of mice of enzyme-treated extract PTBX

Hairs of the back of Balb/c mice (6-8 week-old) were shaved, and the mice were divided into four groups which were the naive group, the control group, the imiquimod (IMQ) + vehicle group and the experimental group, respectively. In the naive group, no treatment is applied to the mice. In the control group, 50 mg imiquimod (IMQ) cream (Aldara; 3M Pharmaceuticals) was smeared on the backs of the mice, and the imiquimod cream was administered once time a day for 6 continuous days to induce parapsoriasis conditions on the mouse skin. In the imiquimod (IMQ) + vehicle group, 50 mg imiquimod cream (Aldara; 3M Pharmaceuticals) was smeared on the backs of the mice, and then the vehicle of ointment was topically smeared, and the imiquimod cream and the vehicle of ointment were administered once time a day for 6 continuous days. In the experimental group, 50 mg imiquimod cream (Aldara; 3M Pharmaceuticals) was smeared on the backs of mice, and then the PTBX ointment was topically smeared (50 mg), and the imiquimod cream and the PTBX ointment were administered once time a day for 6 continuous days.
After that, inflammation level of back skin of the mice was scored. Scoring item for the skin inflammation level comprises erythema and scaling. 0 score (no symptom) to 4 scores (serious) were assigned to back skin of the mice based on the severity of erythema and scaling, and the scores of erythema and scaling were summed up to calculate cumulative scores. The erythema score, scaling score and cumulative score of back skin of the mice of each group are shown in FIGS. 1A, 1B and 1C, respectively.

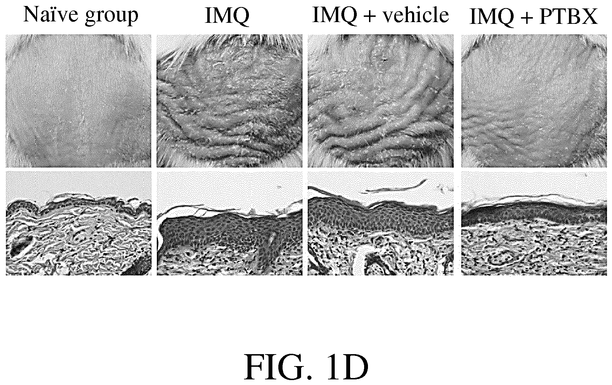

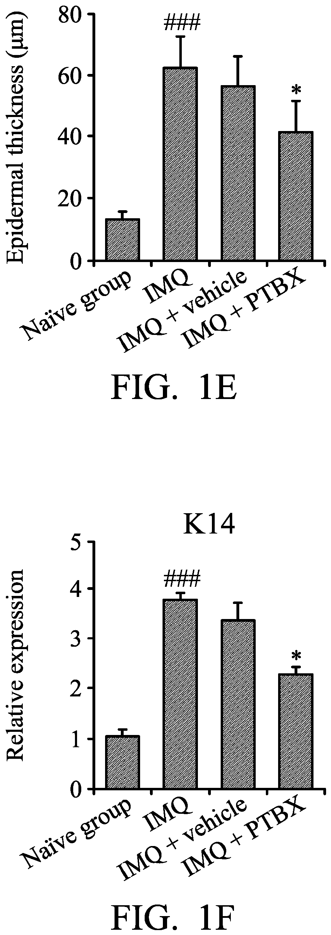

Before sacrificing, the psoriasis nidus of back skin of the mice were photographed and recorded, and after sacrificing, back skin of the mice was sliced, the epidermal thickness thereof was measured, and hematoxylin and eosin (H&E) stain was performed thereon. The photographs and H&E stain results for the back skin of the mice of each group and skin thickness of mice of each group are shown in FIGS. 1D and 1E, respectively.

In addition, detections of gene expression levels of keratin (K14) and filaggrin (FLG) were also performed on parts of tissue of the back skin of the mice, and the results are shown in FIGS. 1F and 1G.

FIGS. 1A-1E show that in the control group, administration of 50 mg imiquimod can result in erythema, scaling and increase of skin thickness of back skin of the mice, however, in the experimental group, by smearing 50 mg PTBX ointment, erythema and scaling of skin can be alleviated and skin thickness can be reduced.

For gene expression level, FIGS. 1F and 1G show that as compared to the naive group, K14 expression of the control group significantly increases (###$p<0.001$), however, smearing 50 mg PTBX ointment has the effect of inhibiting K14 expression (*$p<0.05$). On the contrary, imiquimod results in decrease of FLG expression (#$p<0.05$) while smearing 50 mg PTBX ointment has a protection activity of maintaining FLG expression (**$p<0.01$). Experimental results are shown as Mean $\pm$ Standard deviation.

Example 7

Investigation of enzyme treatment process: Ratio of enzyme to extract

About 1 g dry powder of the crude extract PTB1 was re-dissolved with 50 ml sodium acetate buffer (pH 4.5), and then 0.1, 0.5, 1, 2 or 5 mg $\beta$-glucosidase was added therein, respectively. Next, the enzyme-containing solution was placed in a 37 °C incubator to perform the reaction for 16-24 hours. After the reaction was completed, the solution was placed in a 4°C refrigerator for 24 hours to promote the production of precipitate.

Solid-liquid separation was performed on the solution to take the precipitate out and the precipitate was re-dissolved with methanol to from a mixture solution. After that, high-performance liquid chromatography was performed on the mixture solution, and the results are shown in Table 3.

Table 3: Investigation of ratio condition of enzyme to extract

| Amount of added enzyme | Ratio of weight of added enzyme to the weight of the original herb material (Ratio of the weight of the crude extract PTB1 to the weight of the original herb material is about 1: 1.5-4) | Total content of the four ingredients (mg/g) |
|---|---|---|
| 0.1 mg | 1:15000-40000 | ND |
| 0.5 mg | 1:3000-8000 | 346.26 |
| 1 mg | 1:1500-4000 | 327.85 |
| 2 mg | 1:750-2000 | 317.58 |
| 5 mg | 1:300-800 | 309.25 |
| ND: Not detected | | |

Based on the results shown in Table 3, it is known that, while 0.1 mg enzyme cannot produce precipitate, other amounts of enzyme all have the effect of producing precipitate.

Example 8

Investigation of enzyme treatment process: A variety of enzyme

About 1 g dry powder of crude extract PTB1 was re-dissolved with 50 ml sodium acetate buffer (pH 4.5), and then 1 mg $\beta$-glucosidase, $\alpha$-galactosidase or $\beta$-glucuronidase was added therein, respectively. The ratio of the weight of the added enzyme to the weight of the original herb material was about 1:1500-4000 (the ratio of the weight of the crude extract PTB1 to the weight of the original herb material was about 1:1.5-4). Next, the enzyme-containing solution was placed in a 37°C incubator to perform the reaction for 16-24 hours. After the reaction was completed, the solution was placed in a 4°C refrigerator for 24 hours to promote the production of precipitate.

The solution was centrifuged to isolate the precipitate and the precipitate was re-dissolved with methanol to from a mixture solution. After that, high-performance liquid chromatography was performed on the mixture solution to compare the indicator content difference before and after the enzyme treatment, and the results are shown in Table 4.

Table 4: Indicator content analysis for conversion results of different enzymes

| Sample | Luteolin 7-O-glucoside relative content (%) | Apigenin-7-O-glucoside relative content (%) | Luteolin relative content (%) | Apigenin relative content (%) |
|---|---|---|---|---|
| PTB1 | 100 | 100 | 100 | 100 |
| PTB1+β-glucosidase | 52.7 | 2.8 | 199.3 | 949.4 |
| PTB1+α-galactosidase | 95.3 | 96.2 | 86.6 | 94.7 |
| PTB1+β-glucuronidase | 76.9 | 99.5 | 163.8 | 145.0 |

According to the results of Table 4, it is known that both of β-glucosidase and β-glucuronidase are capable of increasing contents of luteolin and apigenin in the extract of *Chrysanthemum morifolium* through bio-conversion.

Example 9

Investigation of enzyme treatment process: Combination of enzymes

About 1 g dry powder of crude extract PTB1 was re-dissolved with 50 ml sodium acetate buffer (pH 4.5), and then 1 mg β-glucosidase and 1 mg α-galactosidase, or 1 mg β-glucosidase and 1 mg β-glucuronidase were added therein at the same time. The ratio of the weight of the added enzyme to the weight of the original herb material was about 1:750-2000 (the ratio of the weight of the crude extract PTB1 to the weight of the original herb material was about 1:1.5-4). Next, the enzyme-containing solution was placed in a 37°C incubator to perform the reaction for 16-24 hours. After the reaction was completed, the solution was placed in a 4°C refrigerator for 24 hours to promote the production of precipitate.

The solution was centrifuged to isolate the precipitate and the precipitate was re-dissolved with methanol to from a mixture solution. After that, high-performance liquid chromatography was performed on the mixture solution to compare the indicator content difference before and after the enzyme treatment, and the results are shown in Table 5.

Table 5: Indicator content analysis for conversion results of different enzymes

| Sample | Luteolin 7-O-glucoside relative content (%) | Apigenin-7-O-glucoside relative content (%) | Luteolin relative content (%) | Apigenin relative content (%) |
|---|---|---|---|---|
| PTB1 | 100 | 100 | 100 | 100 |
| PTB1+β-glucosidase+α-galactosidase | 35.5 | 2.0 | 194.9 | 897.7 |
| PTB1+β-glucosidase+β-glucuronidase | 37.4 | 9.3 | 282.0 | 990.5 |

According to the results of Table 5, it is known that when β-glucosidase and β-glucuronidase are present at the same time, conversions of luteolin 7-O-glucoside and apigenin-7-O-glucoside to respective luteolin and apigenin can be raised.

Example 10

Investigation of enzyme treatment process: Different plants belonging to *Chrysanthemum*

*Chrysanthemum indicum* L, *Chrysanthemum cinerariifolium* or *Chrysanthemum indicum* and 15 times its weight in water were mixed to form a mixture. The mixture is heated to ebullition (about 90-100°C) to perform a heating extraction for 1 hour, and then solid residues therein were removed to obtain an extract solution.

After the extract solution was cooled to room temperature, an enzyme, β-glucosidase, was added therein, and the ratio of the weight of the added enzyme to the weight of the original herb material was about 1:1500. Next, the enzyme-containing extract solution was place in a 37°C incubator to perform reaction for 16-24 hours.

After the reaction was completed, the enzyme-reacted extract solution was placed in a 4°C refrigerator for 24 hours to promote the production of precipitate.

After that, the extract solution was centrifuged and high-performance liquid chromatography was performed on the supernatant and the precipitate to determine whether other plants belonging to *Chrysanthemum* can also achieve the effects of precipitation through enzyme treatment or not, and an analysis of for *in vitro* keratinocyte proliferation inhibiting activity was performed on the precipitate, and the results are shown in Table 6.

Table 6: Activity analysis results of enzyme-conversion process for plants belonging to *Chrysanthemum*

| | Keratoplasia $GI_{50}$ ($\mu$g/ml) | Whether effects of precipitation purification occur? |
|---|---|---|
| *Chrysanthemum indicum* L | >200 | No |
| *Chrysanthemum indicum* L+$\beta$-glucosidase | >200 | No |
| *Chrysanthemum cinerariifolium* | 50-100 | No |
| *Chrysanthemum cinerariifolium*+$\beta$-glucosidase | 100-200 | No |
| *Chrysanthemum indicum* | >100 | No |
| *Chrysanthemum indicum*+$\beta$-glucosidase | >200 | No |

According to the results of Table 6, it is known that other plants belonging to *Chrysanthemum,* such as *Chrysanthemum indicum* L, *Chrysanthemum cinerariifolium* and *Chrysanthemum indicum,* all cannot be precipitated and purified through the enzyme treatment and keratinocyte proliferation inhibiting activity thereof cannot be raised through the enzyme treatment.

It will be apparent to those skilled in the art that various modifications and variations can be made to the disclosed embodiments. It is intended that the specification and examples be considered as exemplary only, with the true scope of the disclosure being indicated by the following claims and their equivalents.

**Claims**

1. A method for preparing an extract of *Chrysanthemum morifolium* with an effect of treating skin diseases, comprising:

    (a) performing an extraction procedure on *Chrysanthemum morifolium* with an extraction solvent to obtain an extract solution; and
    (b) adding at least one glycoside hydrolase to the extract solution to make an enzyme reaction occur and increase contents of luteolin and apigenin in the extract solution to produce a precipitate,
    wherein the at least one glycoside hydrolases is at least one selected from a group consisting of:

    glucosidase;
    galactosidase; and
    glucuronidase, and

    wherein the precipitate is the extract of *Chrysanthemum morifolium* with an effect of treating skin diseases.

2. The method for preparing an extract of *Chrysanthemum morifolium* with an effect of treating skin diseases according to claim 1, wherein the extract solution comprises water or alcohols, and/or wherein a temperature for the extraction procedure is 70-100°C.

3. The method for preparing an extract of *Chrysanthemum morifolium* with an effect of treating skin diseases according to claim 1or 2, wherein time required for the extraction procedure is 1-3 hours.

4. The method for preparing an extract of *Chrysanthemum morifolium* with an effect of treating skin diseases according to claim 1, wherein the at least one glycoside hydrolases is glucosidase, and the glucosidase is β-glucosidase, or wherein the at least one glycoside hydrolases is glucosidase and glucuronidase, and the glucosidase is β-glucosidase and the glucuronidase is β-glucuronidase.

5. The method for preparing an extract of *Chrysanthemum morifolium* with an effect of treating skin diseases according to any one of claims 1-4, wherein the weight ratio of the at least one glycoside hydrolases to the *Chrysanthemum morifolium* is about 1:200-10000, and/or wherein the temperature of the enzyme reaction is about 25-45°C.

6. The method for preparing an extract of *Chrysanthemum morifolium* with an effect of treating skin diseases according to any one of claims 1-5, wherein the time required for the enzyme reaction is about 5-30 hours, and/or wherein the extract solution is water.

7. The method for preparing an extract of *Chrysanthemum morifolium* with an effect of treating skin diseases according to claim 6, wherein the temperature of the extraction procedure is 70-100°C.

8. The method for preparing an extract of *Chrysanthemum morifolium* with an effect of treating skin diseases according to claim 6, wherein the at least one glycoside hydrolases is β-glucosidase.

9. The method for preparing an extract of *Chrysanthemum morifolium* with an effect of treating skin diseases according to claim 8, wherein the weight ratio of the β-glucosidase to the *Chrysanthemum morifolium* is 1:200-10000.

10. The method for preparing an extract of *Chrysanthemum morifolium* with an effect of treating skin diseases according to claim 6, wherein the at least one glycoside hydrolases is β-glucosidase and β-glucuronidase.

11. The method for preparing an extract of *Chrysanthemum morifolium* with an effect of treating skin diseases according to claim 10, wherein a weight ratio of the β-glucosidase and the β-glucuronidase to the *Chrysanthemum morifolium* is about 1:200-10000, or wherein a weight ratio of the β-glucosidase to the β-glucuronidase is 1-2:1-2.

12. An extract of *Chrysanthemum morifolium* with an effect of treating skin diseases, which is obtained using the method for preparing an extract of *Chrysanthemum morifolium* according to any one of claims 1-11.

13. An extract of *Chrysanthemum morifolium* with an effect of treating skin diseases, at least comprising the following two indicator ingredients:

   luteolin; and
   apigenin,
   wherein in the extract of *Chrysanthemum morifolium,* the sum of the contents of luteolin and apigenin is 15-85 wt%.

**Patentansprüche**

1. Verfahren zum Herstellen eines Extrakts von Chrysanthemum morifolium mit einer Wirkung zur Behandlung von Hautkrankheiten, mit den Schritten:

   (a) Ausführen eines Extraktionsverfahrens an Chrysanthemum morifolium mit einem Extraktionslösungsmittel zum Erhalten einer Extraktlösung; und
   (b) Zugeben mindestens einer Glykosidhydrolase zur Extraktlösung, um zu veranlassen, dass eine Enzymreaktion stattfindet und der Gehalt an Luteolin und Apigenin in der Extraktlösung erhöht wird, zum Erzeugen eines Präzipitats, wobei die mindestens eine Glykosidhydrolase mindestens eine Glykosidhydrolase ist, die ausgewählt ist aus einer Gruppe bestehend aus:

   Glucosidase;
   Galactosidase; und
   Glucuronidase, und
   wobei das Präzipitat der Extrakt von Chrysanthemum morifolium mit einer Wirkung zur Behandlung von Hautkrankheiten ist.

**2.** Verfahren nach Anspruch 1, wobei die Extraktlösung Wasser oder Alkohole aufweist, und/oder wobei eine Temperatur für das Extraktionsverfahren 70 - 100°C beträgt.

**3.** Verfahren nach Anspruch 1 oder 2, wobei die für das Extraktionsverfahren erforderliche Zeit 1 bis 3 Stunden beträgt.

**4.** Verfahren nach Anspruch 1, wobei die mindestens eine Glycosidhydrolase Glucosidase ist und die Glucosidase β-Glucosidase ist, oder wobei die mindestens eine Glycosidhydrolase Glucosidase und Glucuronidase ist, und wobei die Glucosidase β-Glucosidase ist und die Glucuronidase β-Glucuronidase ist.

**5.** Verfahren nach einem der Ansprüche 1 bis 4, wobei das Gewichtsverhältnis der mindestens einen Glycosidhydrolase zu Chrysanthemum morifolium etwa 1:200 - 10000 beträgt, und/oder wobei die Temperatur der Enzymreaktion etwa 25 - 45°C beträgt.

**6.** Verfahren nach einem der Ansprüche 1 bis 5, wobei die für die Enzymreaktion erforderliche Zeit etwa 5 bis 30 Stunden beträgt und/oder wobei die Extraktlösung Wasser ist.

**7.** Verfahren nach Anspruch 6, wobei die Temperatur des Extraktionsverfahrens 70 - 100°C beträgt.

**8.** Verfahren nach Anspruch 6, wobei die mindestens eine Glycosidhydrolase β-Glucosidase ist.

**9.** Verfahren nach Anspruch 8, wobei das Gewichtsverhältnis der β-Glucosidase zu Chrysanthemum morifolium 1:200 - 10000 beträgt.

**10.** Verfahren nach Anspruch 6, wobei die mindestens eine Glycosidhydrolase β-Glucosidase und β-Glucuronidase ist.

**11.** Verfahren nach Anspruch 10, wobei ein Gewichtsverhältnis der β-Glucosidase und der β-Glucuronidase zu Chrysanthemum morifolium etwa 1:200 - 10000 beträgt, oder wobei ein Gewichtsverhältnis der β-Glucosidase zur β-Glucuronidase 1 - 2:1 - 2 beträgt.

**12.** Extrakt von Chrysanthemum morifolium mit einer Wirkung zur Behandlung von Hautkrankheiten, der unter Verwendung des Verfahrens zum Herstellen eines Extrakts von Chrysanthemum morifolium nach einem der Ansprüche 1 bis 11 erhalten wird.

**13.** Extrakt von Chrysanthemum morifolium mit einer Wirkung zur Behandlung von Hautkrankheiten, der mindestens die folgenden zwei Indikatorbestandteile enthält:

Luteolin; und
Apigenin,
wobei im Extrakt von Chrysanthemum morifolium die Summe der Gehalte an Luteolin und Apigenin 15 bis 85 Gew.-% beträgt.


**Revendications**

**1.** Procédé de préparation d'un extrait de *Chrysanthemum morifolium* possédant un effet de traitement de maladies de la peau comprenant :

(a) l'exécution d'une procédure d'extraction sur le *Chrysanthemum morifolium* avec un solvant d'extraction afin d'obtenir une solution d'extrait ; et
(b) l'addition d'au moins une glycoside hydrolase à la solution d'extrait pour provoquer une réaction enzymatique et augmenter les teneurs de lutéoline et d'apigénine dans la solution d'extrait afin de produire un précipité, dans lequel l'au moins une glycoside hydrolase est au moins une hydrolase choisie dans un groupe constitué de :

la glucosidase ;
la galactosidase ; et
la glucuronidase ; et

dans lequel le précipité est l'extrait de *Chrysanthemum morifolium* possédant un effet de traitement de maladies de la peau.

2. Procédé de préparation d'un extrait de *Chrysanthemum morifolium* possédant un effet de traitement de maladies de la peau selon la revendication 1, dans lequel la solution d'extrait comprend de l'eau ou des alcools, et/ou dans lequel une température pour la procédure d'extraction est de 70 à 100 °C.

3. Procédé de préparation d'un extrait de *Chrysanthemum morifolium* possédant un effet de traitement de maladies de la peau selon la revendication 1 ou la revendication 2, dans lequel la durée nécessaire pour la procédure d'extraction est de 1 à 3 heures.

4. Procédé de préparation d'un extrait de *Chrysanthemum morifolium* possédant un effet de traitement de maladies de la peau selon la revendication 1, dans lequel l'au moins une glycoside hydroxylase est la glucosidase, et la glucosidase est la β-glucosidase, ou dans lequel l'au moins une glycoside hydrolase est la glucosidase et la glucuronidase, et la glucosidase et la glucuronidase est la β-glucuronidase.

5. Procédé de préparation d'un extrait de *Chrysanthemum morifolium* possédant un effet de traitement de maladies de la peau selon l'une quelconque des revendications 1 à 4, dans lequel le ratio en poids de l'au moins une glycoside hydrolase sur le *Chrysanthemum morifolium* est d'environ 1 : 200 à 10000, et/ou dans lequel la température de la réaction enzymatique est environ 25 à 45 °C.

6. Procédé de préparation d'un extrait de *Chrysanthemum morifolium* possédant un effet de traitement de maladies de la peau selon l'une quelconque des revendications 1 à 5, dans lequel la durée nécessaire pour la réaction enzymatique est environ de 5 à 30 heures, et/ou dans lequel la solution d'extrait est de l'eau.

7. Procédé de préparation d'un extrait de *Chrysanthemum morifolium* possédant un effet de traitement de maladies de la peau selon la revendication 6, dans lequel la température de la procédure d'extraction est de 70 à 100 °C.

8. Procédé de préparation d'un extrait de *Chrysanthemum morifolium* possédant un effet de traitement de maladies de la peau selon la revendication 6, dans lequel l'au moins une glycoside hydrolase est la β-glucosidase.

9. Procédé de préparation d'un extrait de *Chrysanthemum morifolium* possédant un effet de traitement de maladies de la peau selon la revendication 8, dans lequel le ratio en poids de la β-glucosidase sur le *Chrysanthemum morifolium* est d'environ 1 : 200 à 10000.

10. Procédé de préparation d'un extrait de *Chrysanthemum morifolium* possédant un effet de traitement de maladies de la peau selon la revendication 6, dans lequel l'au moins une glycoside hydrolase est la β-glucosidase et la β-glucuronidase.

11. Procédé de préparation d'un extrait de *Chrysanthemum morifolium* possédant un effet de traitement de maladies de la peau selon la revendication 10, dans lequel le ratio en poids de la β-glucosidase et de la β-glucuronidase sur le *Chrysanthemum morifolium* est d'environ 1 : 200 à 10000, ou dans lequel le ratio en poids de la β-glucosidase sur la β-glucuronidase est de 1 à 2 : 1 à 2.

12. Extrait de *Chrysanthemum morifolium* possédant un effet de traitement de maladies de la peau qui est obtenu en utilisant le procédé de préparation d'un extrait de *Chrysanthemum morifolium* selon l'une quelconque des revendications 1 à 11.

13. Extrait de *Chrysanthemum morifolium* possédant un effet de traitement de maladies de la peau comprenant au moins les deux ingrédients indicateurs suivants :

la lutéoline ; et
l'apigénine,
dans lequel, dans l'extrait de *Chrysanthemum morifolium,* la somme des teneurs en lutéoline et apigénine est de 15 à 85 % en poids.

FIG. 1A

FIG. 1B

FIG. 1C

Naïve group    IMQ    IMQ + vehicle  IMQ + PTBX

FIG. 1D

FIG. 1E

FIG. 1F

FIG. 1G

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103610725 A

- CN 105175376 A

**Non-patent literature cited in the description**

- **XINYAN LU et al.** Evaluation of hepatic clearance and drug-drug interactions of luteolin and apigenin by using primary cultured rat hepatocytes. *Die Pharmazie, Germany,* 604
- Chrysanthemum morifolium Ramat. reduces the oxidized LDL-induced expression of intercellular adhesion molecule-1 and E-selectin in human umbilical vein endothelial cells. **LII C K et al.** JOURNAL OF ETHNOPHARMACOLOGY. ELSEVIER IRELAND LTD, vol. 128, 213-220

- **FU YU-JIE et al.** Enzyme assisted extraction of luteolin and apigenin from pigeonpea [Cajanuscajan(L.) Millsp.] leaves. FOOD CHEMISTRY, ELSEVIER LTD, 07 April 2008, vol. 111, 508-512